Europäisches Patentamt

European Patent Office

Office européen des brevets

(19)

(11) Veröffentlichungsnummer: **0 190 093**
**B1**

(12) **EUROPÄISCHE PATENTSCHRIFT**

(45) Veröffentlichungstag der Patentschrift: **03.10.90**

(51) Int. Cl.⁵: **A 61 F 2/34**

(21) Anmeldenummer: **86730005.5**

(22) Anmeldetag: **10.01.86**

(54) **Künstliche Hüftpfanne mit einem Tragelement und einem Pfanneneinsatz.**

(30) Priorität: **11.01.85 DE 8500869 u**

(43) Veröffentlichungstag der Anmeldung:
**06.08.86 Patentblatt 86/32**

(45) Bekanntmachung des Hinweises auf die
Patenterteilung:
**03.10.90 Patentblatt 90/40**

(84) Benannte Vertragsstaaten:
**BE CH DE FR GB IT LI NL**

(56) Entgegenhaltungen:
**EP-A-0 065 482**
**EP-A-0 066 092**
**DE-A-3 306 151**
**DE-B-2 903 366**
**FR-A-2 519 545**
**GB-A-1 573 608**
**US-A-3 863 273**
**US-A-4 180 873**

(73) Patentinhaber: **MECRON MEDIZINISCHE
PRODUKTE GMBH
Nunsdorfer Ring 23-27
D-1000 Berlin 48 (DE)**

(72) Erfinder: **Anapliotis, Emmanuel
Kiebitzweg 5
D-1000 Berlin 33 (DE)**

(74) Vertreter: **Christiansen, Henning, Dipl.-Ing.
Patentanwalt CHRISTIANSEN Pacelliallee 43/45
D-1000 Berlin 33 (DE)**

Courier Press, Leamington Spa, England.

**Beschreibung**

Die Erfindung betrifft ein Hüftpfannensystem mit Tragelementen und Pfanneneinsätzen der im Oberbegriff des Anspruchs 1 angegebenen Art.

Eine aus der EP-A-65 482 bekannte Hüftpfanne weist einen umlaufenden Sprengring auf, welcher in einander benachbarte entsprechende Aussparungen des Tragelements und der inneren Kunststoffpfanne gleichermaßen eingreift und somit eine axiale Verschiebung in beiden Richtungen verhindert. Dieser Ring muß axial genügend genau geführt sein, damit er den erwünschten Schnappeffekt ermöglicht. Dabei besteht der Nachteil, daß dieser Ring dazu beiträgt, die bei Belastung der Pfanne auftretenden Kräfte vom Pfanneneinsatz auf das Tragelement überzuleiten. Da bei der bekannten Pfanne der Pfanneneinsatz zudem einen unteren überstehenden Kragen aufweist, der am Rand des ringförmigen Tragelements anliegt, sind die Verhältnisse der Kraftüberleitung vom Pfanneneinsatz zum Tragelement nicht unter allen Umständen definiert. Darüberhinaus besteht der Nachteil, daß der Sprengring ein verlierbares Element bildet.

Aus der EP-A 00 066 092 ist eine Prothesenschale für eine Geenkpfanne bekannt, bei der ein pfanneneinsatz mittels eines umlaufenden Vorsprungs in ein die Außenpfanne bildendes Trägerelement einschnappbar ist. Bei dieser bekannten Anordnung bestehen jedoch die Außen- und die Innenschale bevorzugt aus einem gewebeverträglichen Kunststoff, wobei zwischen die beiden Schalen eine dämpfende Pufferschicht eingefügt ist. Wenn die elastischen Dämpfungseigenschaften der Pufferschicht zur Geltung kommen sollen, muß zwischen der Innen- und Außenschale ein radiales Spiel bestehen, welches seinerseits das notwendige erhebliche radiale Übermaß der Einrastmittel bestimmt. Da die gesamte Innenschale seitlich ausweichen kann, besteht die Gefahr, daß die Rastmittel aus der sie aufnehmenden Ausnehmung herausgeraten und somit die stabile Einrastung der Innenpfanne nicht mehr gewährleistet ist. Diese kann in ausgerastetem Zustand bei einem nachfolgenden Lastwechsel in eine undefinierte Lage geraten. Das radiale Übermaß muß auch deswegen groß sein, weil bei einer aus Kunststoff bestehenden Teilekombination den beidseitigen Verformungen unter Belastung Rechnung getragen werden muß.

Weiterhin ist aus der US-A-4 180 873 eine Hüftpfanne bekannt, bei der ein Pfanneneinsatz mittels einer stufenförmigen Aussparung an seiner Randaußenseite hinter einen der Stufung entsprechenden ringförmigen Vorsprung an der Innenseite des Tragelementes einschnappbar ist. Die Außenfläche des Pfanneneinsatzes stellt dabei ein genaues Abbild der Innenfläche des Tragelementes dar. Eine solche ganzflächige Auflage führt aber nur bei absoluter Paßform zu einer gleichmäßigen Krafteinleitung vom Pfanneneinsatz in das Tragelement. Durch die in der obengenannten Druckschrift vorgeschlagene im wesentlichen kegelstumpfartige Außenform des Pfanneneinsatzes bzw. Innenform des Tragelementes wird zudem ohnehin eine gleichmäßige Krafteinleitung von der Hüftpfanne in den umgebenden Knochen ausgeschlossen.

Eine Hüftpfanne mit einem im wesentlichen kugelschalenartig geformten Pfanneneinsatz sowie einem diesen flächenbündig umschließenden ebenfalls kugelschalenartig geformten Tragelement geht aus der FR-A-2 519 545 hervor. Auch hier besteht das Problem der Paßform, die infolge der Elastizität und der Gleichmäßigkeit der Wanddicken bei einseitiger Belastung wenig dauerhaft ist.

Der im kennzeichnenden Teil des Anspruchs 1 angegebenen Erfindung liegt die Aufgabe zugrunde, für ein Tragelement, welches in seinem oberen Innenbereich bereits eine kegelförmige oder sich anderweitig verjüngende Anlagefläche zur Krafteinleitung besitzt, Pfanneneinsätze zu finden, bei denen die Kraftübertragung zum Tragelement in präzise vorherbestimmbaren Bereichen derart erfolgt, daß die Schnappverbindung zwischen beiden Elementen ohne Verwendung zusätzlicher Teile möglichst einfach gehalten werden kann und möglichst geringen Belastungen bzw. Bewegungen unterliegt. Dabei soll insbesondere im Hinblick auf unterschiedliche Belastungsformen und -Größenordnungen eine individuelle Auswahl von Pfanneneinsätzen bei unkomplizierter Austauschbarkeit gegeben sein.

Durch die erfindungsgemäße Gestaltung der Pfanneneinsätze sind diese innerhalb des Tragelementes zentrierbar und trotzdem leicht austauschbar.

Die Einleitung der maximalen Druckbelastungen erfolgt im Bereich der konischen Anlagefläche unter gleichzeitiger Zentrierung. Eine zusätzliche Zentrierung erfolgt im Bereich der zylindrischen Flächen im Bereich des Randes nahe der Öffnung zum Einsetzen des Pfanneneinsatzes. Damit wird der Einsatz einerseits gegen radiale Verschiebungen gesichert und ist andererseits auch nicht verkantbar, so daß ein relativ schmaler Kragen bzw. ein geringfügiges Übermaß im Randbereich ausreichend ist, um den Einsatz zu sichern.

Gleichzeitig nimmt der Pfanneneinsatz elastische Verformungen auf, so daß schockartige Belastungen gut abgefangen werden können. Dadurch, daß der Pfanneneinsatz aus Kunststoff besteht, ist insbesondere auch durch das vorzugsweise zu verwendende Polyäthylenmaterial, für eine ausreichende Dämpfung gesorgt. Der metallene Außenring bewirkt, daß insbesondere die Eingriffsverhältnisse im Acetabelum stabil und auch die Anlageflächen für die Innenpfanne unter Belastung geometrisch genügend genau erhalten bleiben.

Durch die exakte Zentrierung der Innenpfanne können die Überstände der Rastmittel so gering gehalten werden, daß ein Ein- und Ausschnappen der Innenpfanne zu Ersatzzwecken auch dann möglich ist, wenn das äußere Tragelement implantiert verbleibt. Darin besteht der wesentliche Vorteil der hier verwendeten Materialkombination mit ·einem äußeren Metallring, der mit selbstschneidender Wirkung in das Acetabulum einbringbar ist und dort fixiert erhalten werden kann. Das ist insbesondere deswegen günstig, weil der Außenring mit der Zeit einwächst und so seine Position zunehmend verfestigt, so daß ein Entfernen mit einer unnötigen Belastung der Patienten und dem Verlust von Knochenmaterial verbunden wäre. Durch den unproblematischen Ersatz des Pfanneneinsatzes gestalten sich Reoperationen einfacher und ein Austausch der Innenpfanne, d.h. des Pfanneneinsatzes ist ohne weiteres möglich, wobei das Tragelement im wesentlichen unverformt bleibt und somit seine Position im Knochen nicht ändert, während die für die Rastung erforderliche Deformation ausschließlich vom Pfanneneinsatz übernommen wird. Das erfindungsgemäße Hüftpfannensystem ermöglicht insbesondere auch den Austausch eines Pfanneneinsatzes, der sich in dem jeweiligen individuellen Fall nicht bewährt hat durch einen andersartig gestalteten Pfanneneinsatz unter Beibehaltung des implantierten Tragelementes.

Da sich die Anlagefläche zur Kraftübertragung von der Innen- auf die Außenpfanne in einem vorderen sich verjüngenden Bereich befindet und sowohl hier als auch in der Nähe des Randbereichs der Öffnung die axiale Zentrierung des Pfanneneinsatzes erfolgt, kann die dazwischen befindliche, die Einschnappmittel aufweisende Kante der Innenpfanne genügend elastisch sein, um die zum Einschnappen erforderlichen Bewegungen ausführen zu können. Bei einer anderen Ausführung des Pfanneneinsatzes sorgt ein geringfügiges Übermaß im Bereich der zylindrischen Fläche in der Nähe des Randbereichs für die notwendige Arretierung. Es versteht sich, daß auch bei einem Verklemmen mittels eines zylindrischen Übermaßes eine definierte Ausrichtung des Einsatzes - wie sie mit den erfindungsgemäßen Maßnahmen erzielt wird - erforderlich ist, damit die auftretenden Haltekräfte einen Austausch des Pfanneneinsatzes zulassen.

Der Erfindung liegt ferner die Erkenntnis zugrunde, daß die Tragrippen einer selbstschneidenden Prothese eine derart stabile Verankerung innerhalb des Acetabulums finden, daß sie genauso wie die Oberseite des Kunststoffpfanneneinsatzes zur Einleitung der das Hüftgelenk belastenden Kräfte in das Acetabulum dienen können. Zumal sich mit der sich verjüngenden Innenfläche des Tragelementes eine Anlagefläche bietet, welche die Kräfte in der Nähe der Tragflanken aufnimmt und sie in den radialen Teil des Ringes überleitet. Für den Chirurgen stehen hierbei - wie erläutert - zwei alternative Ausführungen zur Wahl: entweder wird der aus hochmolekularem Polyäthylen hergestellte Pfanneneinsatz mit einem ringförmigen Einsatz in das Tragelement mit geringfügigem Druck eingepreßt und ist dort - gegen unbeabsichtigtes Herausfallen durch ein Übermaß einer zylinderförmigen Zentrierungsfläche gesichert - axial verschiebbar. Wird eine Art einer lösbaren Verbindung angestrebt, so kann die Hinterschneidung der Zentrierungsfläche des Tragelementes und eine entsprechende Kante mit einer Abschrägung in Einführungsrichtung des Pfanneneinsatzes mittels eines "Schnappeffektes" ermöglichen, daß die pfanne ohne Beschädigung aus dem Tragelement entfernbar ist. In diesem Fall braucht der Einsatz kein Übermaß aufzuweisen.

Bei einer bevorzugten Weiterbildung der Erfindung, die auf einer umlaufenden einschnappbaren Kante als Rastung basiert, weist der in einer Ausnehmung des Trägerelementes einrastende umlaufende Vorsprung des Einsatzes einen zungenartigen Querschnitt auf und grenzt beidseits der Zunge mit einem umlaufenden Einstich an die Außenoberfläche des Einsatzes an. Auf diese Weise ist der vorstehende, die Außenkontur der Innenpfanne überragende Bereich äußerst flexibel und führt zu einem Einsetzen des Innenteils mit geringem Kraftaufwand. Zum anderen kann die Ausnehmung in der Außenpfanne, in die der Raststeg mit zungenartigem Querschnitt einrastet, ebenfalls als Einstich ausgeführt sein, so daß insoweit Übereinstimmung mit herkömmlichen Außenpfannen besteht, die mittels eines einschnappbaren Federringes mit der Innenpfanne verbunden wurden. Auf diese Weise ist die Lagerhaltung wesentlich reduziert.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 ein Tragelement mit eingesetzter Kunststoffpfanne in Seitenansicht,

Figur 2 die selbe Anordnung im Schnitt,

Figur 3a eine vergrößerte Darstellung der Innenkontur des Tragelementes im Detail,

Figuren 3b und c der neuartigen Außenkonturen eines Pfanneneinsatzes,

Figur 4a ein weiteres Ausführungsbeispiel des Tragelementes sowie

Figur 4b einen Pfanneneinsatz, insbesondere im Zusammenwirken mit einem Tragelement gemäß Figur 4a gemäß einer anderen bevorzugten Ausführungsform.

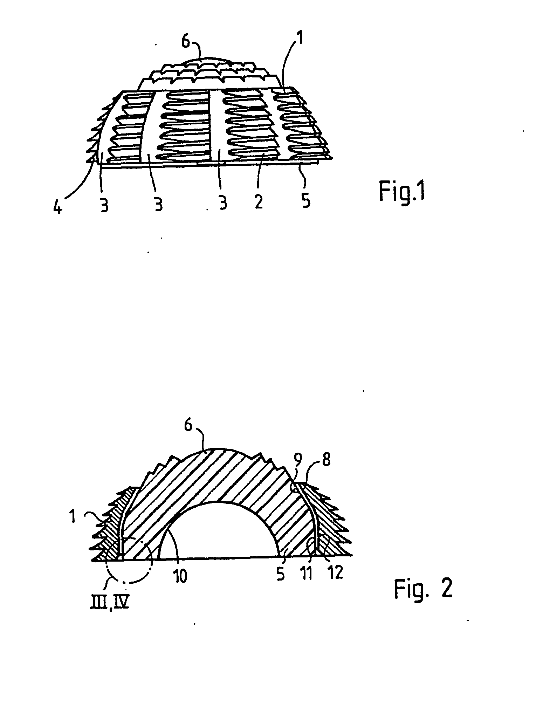

Bei dem in Figur 1 dargestellten Tragelement 1, welches ringförmig ausgebildet und dessen Außenfläche der Oberfläche einer Kugelzone entspricht, sind eine Anzahl von auf dem Umfang verteilten Gewindesegmenten 2 vorgesehen, welche durch Schneidnuten 3 voneinander getrennt sind. Die Gewindesegmente sind an ihren beim Einschrauben vorangeführten Teilen scharfkantig ausgeführt und stehen hier am weitesten von der Kugeloberfläche ab. Sie bilden im Bereich der Schneidnuten scharfkantige Spitzen, welche beispielhaft mit 4 bezeichnet sind. In - bezogen auf die beim Einschrauben aufzuwendende Drehrichtung - weiter zurückliegenden Bereichen sind die Gewindesegmente zu ihren Außenkanten abgestumpft bis abgeflacht ausgebildet, was durch eine parallele Bearbeitung der übereinanderliegenden Gewindesegmente mittels Fräsen oder dgl. erfolgen kann. Das ringförmige Tragelement besteht bevorzugt aus einer Titanlegierung, dessen Elastizitätseigenschaften bei geeigneter Querschnittsbemessung in etwa denen des umgebenden Knochenmaterials entsprechen.

Ein Pfanneneinsatz 5 ist an die inneren Querschnittsabmessungen des Tragelementes 1 angepaßt und läßt sich in das bereits in den Knochen eingeschraubte Tragelement von unten her einsetzen, wobei der obere kuppelförmige Bereich 6 des Einsatzes das Tragelement überragt und sich im eingesetzten Zustand im Inneren der im Knochen angebrachten Aussparung abstützt.

In der Figur 2 sind das Tragelement 1 und ein Pfanneneinsatz 5 im Schnitt prinzipiell wiedergegeben. Die beiden Teile weisen aneinander angepaßte, konisch geformte Bereiche 8 bzw. 9 auf. Die Krafteinleitung unter Belastung erfolgt über die Flanken des kegelstumpfförmigen konischen Bereiches 9 in den eine entsprechend ausgebildete Ausnehmung bildenden Bereich 8 des Tragelements 1. Der in Figur 2 wiedergegebene Einsatz 5 weist an seiner Unterseite eine Ausnehmung 10 zur Aufnahme des Kopfes der Femurschaftprothese auf. Die zylindrische Außenfläche 11 am Tragelement 1 und die zylindrische Innenfläche 12 am Pfanneneinsatz 5 sorgen ebenfalls für eine axiale Zentrierung. Es ist ersichtlich, daß dadurch, daß die Fläche 8 und 9 Zentrierung und eine Wegbegrenzung für den Einsatz bilden, zusammen mit den zylindrischen Flächen 11 und 12 von geringer Höhe eine definierte Führung der beiden Teile 1 und 5 relativ zueinander erfolgt. Die in den nachfolgend zu beschreibenden Figuren wiedergegeben Details befinden sich innerhalb des strichpunktiert umrandeten Kreises III, IV.

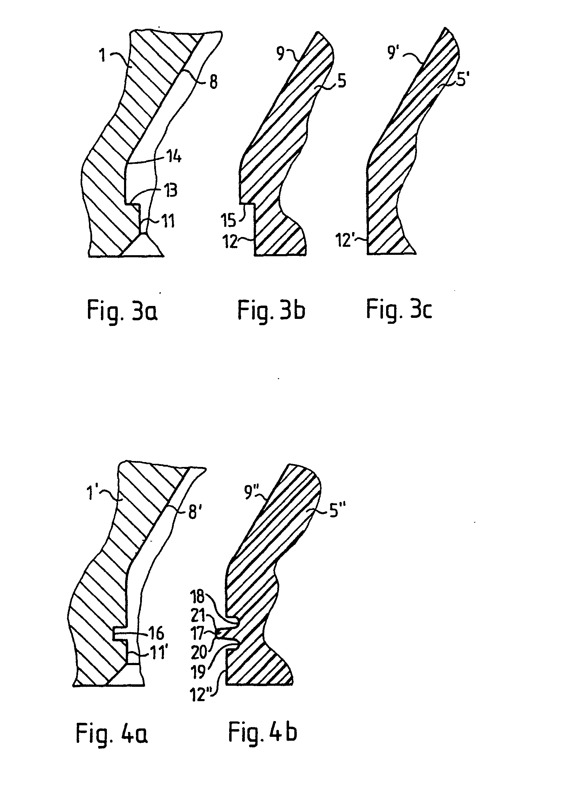

Die Figuren 3a bis 3c zeigen Teilschnitte der rotationssymmetrischen Körper, wobei die Schnittebene innerhalb einer radialen Ebene gelegen ist. In diesen Figuren ist lediglich der Bereich der Kontaktfläche zwischen Tragelement und innerer Pfanne dargestellt. In Figur 3a ist der Bereich der Innenfläche des Tragelementes 1 im Schnitt wiedergegeben, wobei eine Fläche 8 eine sich verjüngende Anschlagfläche für die Pfanne bildet, welche zur Übertragung der im implantierten Zustand auf die Pfanne wirkenden Kräfte auf das Tragelement dient. An die Fläche 8 schließt eine Stufe 13 an, welche eine Hinterschneidung für eine Fläche 11 bildet, die zylindrisch ausgebildet ist. Zwischen Hinterschneidung 13 und der Anschlagfläche 8 befindet sich eine ebenfalls im wesentlichen zylindrische Fläche 14, welche eine axiale Verschiebung eines hinter die Hinterschneidung eingreifenden Halteelements (umlaufender Vorsprung 15 in Figur 3b) ermöglicht.

In Figur 3b ist der Schnitt der Außenkonturen eines ersten Pfanneneinsatzes 5 wiedergegeben, dessen äußere Fläche 9 der Innenfläche 8 des Tragelementes 1 angepaßt ist. Während die zylindrische Fläche 12 des pfanneneinsatzes 5 im Hinblick auf die Fläche 11 des Tragelementes 1 ein gewisses Spiel aufweist, ist eine vorspringende Kante 15 derart dimensioniert, daß sie an der entsprechenden Fläche 13 des Tragelementes anliegt, wenn die Pfanne nicht belastet ist. Da im übrigen der Einsatz 5 geringfügig verschiebbar ist, kann sich somit der Kunststoffpfanneneinsatz unter Belastung mit seiner Fläche an die Innenfläche 8 des Tragelementes 1 anlegen und ist durch den Vorsprung 15 am Herausfallen gehindert. Bei Verschiebung in axialer Richtung unter Last bewegt sich der umlaufende Vorsprung 15 entlang der Fläche 14. Durch die Abschrägung der Fläche auch in ihrem unteren Bereich läßt sich ein entsprechend der Figur 3b ausgebildetes Pfanenteil in ein Tragelement gemäß Figur 3a mit leichtem Druck einschnappen.

Es ist ersichtlich, daß die Anlage der Pfanneneinsätze in das Trägerelement gemäß Figuren 3a und b zur Einleitung der unter Belastung entstehenden Druckkräfte in den Bereichen der Flächen 8 und erfolgt, und hier auch eine Zentrierung der Pfanne vorgenommen wird, welche durch die zylindrischen Flächen 11 und 12 noch unterstützt wird, wobei die hauptsächliche Zentrierung über die erstgenannten Flächen erfolgt und die Flächen 11 und 12 dafür sorgen, daß der der Öffnung nahegelegene Bereich des Pfanneneinsatzes mit der umlaufenden Anschlagkante (Vorsprung) 15 in einem so engen Toleranzbereich geführt ist, daß die Breite der Kante 15 nur sehr gering zu sein braucht, so daß die zum Einsetzen des Einsatzes erforderliche Verformung manuell aufgebracht werden kann. Außerdem wird ein Verkanten verhindert. Somit ist es leicht möglich mit manuellem Krafteinsatz den Einsatz 5 wieder zu entfernen. Der entsprechende Überstand des Vorsprungs 15 beträgt ca. 0,4 mm.

Zu berücksichtigen ist auch, daß durch die elastische Gestaltung des Pfanneneinsatzes sich dieser durch Verformung den Implantationsgegebenheiten anpassen kann, wobei in jedem Fall die zylindrischen Flächen 11 und 12 dafür sorgen, daß die überstehende Avertierungskante eingreift.

Bei dem in Figur 3c wiedergegebenen Pfanneneinsatz 5' weist eine an die Fläche 11 des Tragelementes 1 angepaßte zylindrische, einen Bund bildende Fläche 12' ein leichtes Übermaß auf, so daß sich die Pfanne 5' mit ihrer Außenfläche 9' unter Belastung an das Tragelement 1 anlegt und auch - entsprechend der Lasteinwirkung - verschiebbar ist, aber durch das Übermaß des Durchmessers der zylindrischen Fläche 12' nicht herausfällt. Der Einsatz 5' kann aber bei Bedarf jederzeit aus dem Tragelement 1 herausgezogen werden.

Auch bei dem Pfanneneinsatz gemäß Figur 3c wird die Hauptlast von der Fläche 9' auf die entsprechende Fläche 8 des Tragrings 1 übergeleitet. Die zylindrische Fläche 12' weist ein Übermaß auf, wobei hier entsprechend einem Stopfen dafür gesorgt ist, daß der pfanneneinsatz bei den in der Benutzung zu erwartenden Kräften festgehalten wird und auch beim Auftreten von durch den Einsatz 5' verkantenden Belastungen kein Lösen desselben eintreten kann.

Die Figur 4a zeigt eine Ausführung eines Trägerelementes 1' mit einem umlaufenden Einstich 16 mit rechteckigem Querschnitt. Das dabei benutzte Einsatzteil 5'' weist einen im Querschnitt zungenförmigen Vorsprung 17 auf, der beidseitig von Einstichen 18 und 19 begrenzt ist. Der zungenförmige Vorsprung 17 steht nur geringfügig über die Außenkontur des übrigen Pfanneneinsatzes 5'' vor, so daß er mit geringem Kraftaufwand in die Pfanne gemäß Figur 4a eingefügt werden kann. Er ist aber auch mit einem Trägerelement gemäß Figur 3a verwendbar. Die Gestaltung des in das Trägerelement einschnappenden Vorsprungs ist aber derart, daß auch ein mehrfaches Aus- und Einsetzen des Pfanneneinsatzes - entsprechend den medizinischen Notwendigkeiten - möglich ist. Der Vorsprung 17 mit zungenartigem Querschnitt ist bevorzugt abgeschrägt und verjüngt sich in Einführungsrichtung des Pfanneneinsatzes, so daß ein Einschnappen beim Einsetzen begünstigt ist, ein unbeabsichtigtes Herausfallen jedoch verhindert ist.

Bevorzugte Abmessungen sind ca. 0,7 mm für die Breite der Zunge im Querschnitt, ein Radius von 0,5 mm für die Einstiche 18 und 19, welche jeweils einen halbkreisförmigen Querschnitt haben. Der zungenartige Vorsprung 17 steht dabei um ca. 0,4 mm gegenüber der übrigen äußeren Kontur des Pfanneneinsatzes 5'' vor und kann gegebenenfalls im Querschnitt mit einem relativ großen Radius verrundet oder abgeschrägt sein, um das Einschnappen beim Einsetzen der Innenpfanne zu begünstigen. Es ist ersichtlich, daß der zungenartige Vorsprung 17 eine relativ große seitliche Beweglichkeit aufweist, so daß er bei radialer Belastung nahezu vollständig innerhalb der Kontur des Pfanneneinsatzes verschwindet und wieder hervortritt, sobald der Einstich 16 erreicht ist.

Es ist weiterhin ersichtlich, daß die Pfanneneinsätze durch die sich verjüngenden Frontflächen, welche an die entsprechenden Innenflächen der Trägerelemente angepaßt sind, radial zentriert werden, so daß sie koaxial zum Trägerelement ausgerichtet sind. Auf diese Weise ist dafür gesorgt, daß auch unter Belastung der pfanneneinsatz nicht in radialer Richtung auswandert, so daß die einschnappende Kante außer Eingriff mit der sie aufnehmenden Nut bzw. dem entsprechenden Ansatzgerät und der Pfanneneinsatz unerwünschter Weise freikommt. Trotzdem ist der Polyäthyleneinsatz - insbesondere in seinem der Öffnung zugewandten Bereich elastisch verformbar, so daß er auch stoßartig auftretende Kräfte absorbiert. Des weiteren ist günstig, daß auch herkömmliche Elemente verwendbar sind.

Bei dem Ausführungsbeispiel gemäß Figur 4b sorgt die Kante 12'' für die zylindrische Ausrichtung, so daß der vorstehende Ansatz mit Zungenquerschnitt 17 nur geringfügig überstehen muß. Die Kante 20 verhindert - entsprechend der Kante 15 bei dem Ausführungsbeispiel gemäß Figur 3b -, daß der Einsatz aus dem Trägerelement herausgelangen kann. Die gegenüberliegende Kante 21 kann demgegenüber ein gewisses Spiel aufweisen. Bei der Ausführung gemäß 4b gelangt der zungenartige Vorsprung bei manuellem Einsetzen bzw. Her-ausnehmen des Einsatzes durch seine Flexibilität (und ein entsprechendes Umbiegen) in den Bereich der Ausnehmungen bildenden Einstiche 18 bzw. 19, so daß insoweit eine sehr einfache Handhabbarkeit gegeben ist. Der zungenartige Vorsprung 17 bildet eine relativ elastische Arretierung, die nach beiden Seiten auch geringfügig ausweichen kann, so daß auch Verformungen des Trägerelementes bzw. des Pfanneineinsatzs gefolgt werden kann, wobei die Hauptkrafteinleitung ebenfalls im Bereich der Flächen 8 und 9 erfolgt. Durch die doppelte Zentrierung ist ein seitliches Auswandern des Pfanneneinsatzes und ein Verkanten verhindert, so daß die Vorsprünge mit zungenartigem Querschnitt den Einsatz auch bei verhältnismäßig geringer Eingriffstiefe festhalten.

Zum Einfügen und Herausnehmen des pfanneneinsatzes sind keine Spezialwerkzeuge erforderlich, sondern die entsprechenden Vorgänge können direkt manuell ausgeführt werden.

Durch Auswahl des entsprechenden Pfanneneinsatzes und gegebenenfalls durch Austausch von Pfanneneinsätzen zum Beispiel bei Verschleiß oder bei insbesondere altersbedingten Änderungen der Belastungsbedingungen kann der Arzt so der individuellen Tragfähigkeit des Acetabulums des Patienten Rechnung tragen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

**Patentansprüche**

1. Hüftpfannensystem mit einem Tragelement aus Metall und Pfanneneinsätzen aus Kunststoff, wobei das Tragelement aufweist:

eine an der Innenfläche vorgesehene, kreisringförmig geschlossene sich sphärisch oder konisch bzw. in anderer Weise verjüngende Anschlagfläche (8) für den Pfanneneinsatz sowie

eine der Aufnahmeöffnung benachbarte eine zylindrische Innenfläche bildende Zentrierungsfläche (11), welche eine umlaufend Hinterschneidung (13) bzw. einen umlaufenden Einstich (16) aufweist, die mindestens mittelbar an die Anschlagfläche (8) anschließt,

dadurch gekennzeichnet, daß ein erster Pfanneneinsatz (5 oder 5'') vorgesehen ist mit

einem an die Hinterschneidung bzw. den Einstich angepaßten umlaufendem Vorsprung (15, 17) zum Einschnappen des Pfanneneinsatzes (5 oder 5'') in das Trägeelement (1), wobei in bezug auf die Anschlagfläche ein axiales Spiel besteht

sowie daß ein weiterer Pfanneneinsatz (5') vorgesehen ist mit

einer zylindrischen Außenfläche (12) im Randbereich, die angepaßt an die innere Zentrierungsfläche (11), mit einem Übermaß als Klemmsitz versehen ist.

2. Hüftpfannensystem nach Anspruch 1, gekennzeichnet durch mindestens einen Pfanneneinsatz mit einem Vorsprung (15, 20), der eine Abschrägung aufweist, welche sich in Richtung zum Einsetzen des Pfanneneinsatzes (5) verjüngt.

3. Hüftpfannensystem nach einem der vorangehenden Ansprüche, gekennzeichnet durch mindestens einen Pfanneneinsatz mit einem umlaufenden Vorsprung (17), der einen zungenartigen Querschnitt aufweist, wobei auschließlich der Bereich der Zunge die Außenkontur des Pfanneneinsatzes überragt und die Zunge insbesondere eine Breite von 0,7 mm aufweist.

4. Hüftpfannensystem nach einem der vorangehenden Ansprüche, gekennzeichnet durch mindestens einen Pfanneneinsatz mit einem Vorsprung (17), dem mindestens einseitig ein Einstich (18, 19) benachbart ist.

5. Hüftpfannensystem nach Anspruch 4, dadurch gekennzeichnet, daß der Einstich (18, 19) einen Radius von im wesentlichen 0,5 mm aufweist.

6. Hüftpfannensystem nach einem der vorangehenden Ansprüche, dadurch gekennzeichnet, daß ein Anschlag für den Pfanneneinsatz (5, 5' oder 5'') in Richtung zum Einsetzen in das Tragelement (1) im wesentlichen ausschließlich durch die Anlagefläche (8) gebildet wird.

7. Hüftpfannensystem nach einem der vorangehenden Ansprüche, gekennzeichnet durch mindestens einen Pfanneneinsatz mit einem Überstand des Vorsprungs (17), der im wesentlichen 0,4 mm beträgt.

8. Hüftpfannensystem nach einem der vorangehenden Ansprüche, dadurch gekennzeichnet, daß mindestens ein Tragelement (1) aus einer körperverträglichen Titanlegierung besteht.

9. Hüftpfannensystem nach einem der vorangehenden Ansprüche, dadurch gekennzeichnet, daß mindestens ein Pfanneneinsatz (5) aus hochmolekularem Polyäthylen besteht.

## Revendications

1. Système de cupule d'articulation de hanche comprenant un élément porteur en métal et des inserts de cupule en matière plastique, dans lequel l'élément porteur présente

une surface de butée (8), prévue sur la surface intérieure, fermée en forme d'anneau circulaire, qui se rétrécit avec une forme sphérique, conique ou autre, destinée à arrêter l'insert de cupule, ainsi qu'une surface de centrage (11), adjacente à l'ouverture réceptrice, formant une surface intérieure cylindrique, qui présente une contre-dépouille périphérique (13) ou une encoche périphérique (16), qui fait suite au moins indirectement à la surface de butee (8),

caractérisé en ce qu'il est prévu un premier insert de cupule (5 ou 5'') possédant

une saillie (15, 17) périphérique, adaptée à la contre-dépouille ou à l'encoche respectivement, qui sert à encliqueter l'insert de cupule (5 ou 5'') dans l'élément porteur (1), où il subsiste un jeu axial par rapport à la surface de butée,

et en ce qu'il est prévu un autre insert de cupule (5') possédant

une surface extérieure cylindrique (12) située dans la région marginale, qui est adaptée à la surface de centrage intérieure (11) avec une surcote, pour établir un ajustement serré.

2. Système de cupule d'articulation de hanche selon la revendication 1, caractérisée par au moins un insert de cupule présentant une saillie (15, 17) qui présente un chanfrein qui se rétrécit dans le sens de la mise en place de l'insert de cupule (5).

3. Système de cupule d'articulation de hanche selon une des revendications précédentes, caractérisé par au moins un insert de cupule présentant une saillie périphérique (17) à section transversale en forme de languette, dans laquelle seule la région de la languette déborde audelà du profil extérieur de l'insert de cupule et la languette présente en particulier une largeur de 0,7 mm.

4. Système de cupule d'articulation de hanche selon une des revendications précédentes, caractérisé par au moins un insert de cupule possédant une saillie (17) à laquelle une encoche (18, 19) est adjacente au moins sur un côté.

5. Système de cupule d'articulation de hanche selon la revendication 4, caractérisé en ce que l'encoche (18, 19) présente un arrondi d'environ 0,5 mm.

6. Système de cupule d'articulation de hanche selon une des revendications précédentes, caractérisé en ce qu'une butée servant à arrêter l'insert de cupule (5, 5' ou 5'') dans le sens de la mise en place dans l'élément porteur (1) est formé sensiblement exclusivement par la surface d'appui (8).

7. Système de cupule d'articulation de hanche selon une des revendications précédentes, caractérisé par au moins un insert de cupule muni d'un déport de saillie (17) qui représente sensiblement 0,4 mm.

8. Système de cupule d'articulation de hanche selon une des revendications précédentes, caractérisé en ce qu'au moins un élément porteur (1) est composé d'un alliage de titane physiologiquement compatible.

9. Système de cupule d'articulation de hanche selon une des revendications précédentes, caractérisé en ce qu'au moins un insert de cupule (5) est composé de polyéthylène à haut poids moléculaire.

## Claims

1. Hip cup system with a metal support element and plastic cup inserts, in which the support element has:

a spherically or conically or otherwise tapering stop surface (8) for the cup insert, provided on the inner surface and annularly closed, and a centering surface (11) adjacent to the receiving opening, forming a cylindrical inner surface and having a circumferential undercut (13) or a circumferential groove (16) which adjoins the stop surface (8) at least centrally,

characterized in that a first cup insert (5 or 5'') is provided, with a circumferential projection (15, 17) adapted to the undercut or the groove and for snapping the cup insert (5 or 5'') into the support element (1), axial play existing relative to the stop surface,

and in that a further cup insert (5') is provided, with a cylindrical outer surface (12) in the edge region which is provided, adapted to the inner centering surface (11), as an overdimensioned clamping seat.

2. Hip cup system according to Claim 1, characterized by at least one cup insert with a projection (15, 17) which exhibits a slope which tapers in the direction of insertion of the cup insert (5).

3. Hip cup system according to one of the preceding claims, characterized by at least one cup insert with a circumferential projection (17) which has a tongue-like cross-section, only the region of the tongue projecting above the outer contour of the cup insert and the tongue having, in particular, a width of 0.7 mm.

4. Hip cup system according to one of the preceding claims, characterized by at least one cup insert with a projection (17) which is adjacent, on at least one side, to a groove (18, 19).

5. Hip cup system according to Claim 4, characterized in that the groove (18, 19) has a radius of essentially 0.5 mm.

6. Hip cup system according to one of the preceding claims, characterized in that a stop for the cup insert (5, 5' or 5'') is formed in the direction of insertion into the support element (1) essentially only by the stop surface (8).

7. Hip cup system according to one of the preceding claims, characterized by at least one cup insert with a projection (17) which juts out by essentially 0.4 mm.

8. Hip cup system according to one of the preceding claims, characterized in that at least one support element (1) consists of a body-compatible titanium alloy.

9. Hip cup system according to one of the preceding claims, characterized in that at least one cup insert (5) consists of high-molecular polyethylene.

Fig.1

Fig. 2

1

Fig. 3a      Fig. 3b      Fig. 3c

Fig. 4a      Fig. 4b